# EUROPÄISCHE PATENTANMELDUNG

(11) **EP 1 624 075 A2**
(43) Veröffentlichungstag der Anmeldung: **08.02.2006**
(21) Anmeldenummer: 05016029.0
(22) Anmeldetag: 22.02.2001
(51) Int. Cl.: C12Q 1/68

(54) **Nachweis von humanen Papillomviren**

(30) Priorität: 26.02.2000 DE 10009143
(62) Teilanmeldung aus: 01911683.9
(71) Anmelder: Eberhard-Karls-Universität Tübingen, 72076 Tübingen (DE)
(72) Erfinder: Iftner, Thomas, 72145 Hirrlingen (DE)
(74) Vertreter: Otten, Hajo

(57) **Zusammenfassung**

Es wird ein Verfahren zum Nachweis von Humanen Papillomviren (HPV) in biologischem Material beschrieben, bei dem eine Extraktion/Isolation von Nukleinsäuren aus biologischem Material und der Nachweis HPV-spezifischer DNA aus den isolierten Nukleinsäuren über eine Amplifikation spezifischer HPV-DANN-Abschnitte mittels Polymerase-Kettenreaktion (PCR) erfolgt. Dazu wird zumindest ein Oligonukleotid verwendet, welches mit Oligonukleotiden hybridisiert, mit welchen ein Oligonukleotid mit der SEQ ID Nr. 1, SEQ ID Nr. 2 oder SEQ ID Nr. 3 aus dem beiliegenden Sequenzprotokoll hybridisiert und welches Oligonukleotid die gleiche Funktion aufweist wie ein Oligonukleotid mit der SEQ ID Nr. 1, SEQ ID Nr. 2 oder SEQ ID Nr. 3 aus dem beiliegenden Sequenzprotokoll.

SEQUENZPROTOKOLL

<110> Eberhard-Karls-Universität Tübingen

<120> Nachweis von HPV

<130> 5402P183WO

<140> PCT/EP01/02003
<141> 2001-02-22

<160> 5

<170> PatentIn Ver. 2.1

<210> 1
<211> 27
<212> DNA
<213> Künstliche Sequenz

<220>
<223> Beschreibung der künstlichen Sequenz:
      Nukleotidsequenz

<400> 1
aacaatgtgt agacattata aacgagc                                          27

<210> 2
<211> 23
<212> DNA
<213> Künstliche Sequenz

<220>
<223> Beschreibung der künstlichen Sequenz:
      Nukleotidsequenz

<400> 2
attaaactca ttccaaaata tga                                          23

<210> 3
<211> 22
<212> DNA
<213> Künstliche Sequenz

<220>
<223> Beschreibung der künstlichen Sequenz:
      Nukleotidsequenz

<400> 3
gaggytgcaa ccaaaamtgr ct                                           22

<210> 4
<211> 30
<212> DNA
<213> Künstliche Sequenz

<220>
<223> Beschreibung der künstlichen Sequenz: 3'-Sonde für
      den Nachweis von HPV33

<400> 4
cgtaaaatgt caataggaca atggatacaa                                   30

<210> 5

<211> 29

<212> DNA

<213> Künstliche Sequenz

<220>

<223> Beschreibung der künstlichen Sequenz:5'-Sonde für
      den Nachweis von HPV33

<400> 5
agatgtgaaa aaacaaatga tggaggaaa                                    29

## Beschreibung

Die vorliegende Erfindung betrifft ein Verfahren zum Nachweis von Humanen Papillomviren (HPV) in biologischem Material sowie in dem Verfahren verwendete Nukleotidsequenzen.

Das Verfahren umfaßt dabei die Schritte:
a) Extraktion/Isolation von Nukleinsäuren aus biologischem Material, und
b) Nachweis HPV-spezifischer DNA aus den isolierten Nukleinsäuren über eine Amplifikation spezifischer HPV-DNA-Abschnitte mittels Polymerase-Ketten-Reaktion (PCR).

Ein derartiges Verfahren ist aus verschiedenen Veröffentlichungen bekannt.

Infektionen mit dem HPV stehen in Verbindung mit einer Vielzahl von Erkrankungen der Haut und der Schleimhaut. Meist handelt es sich dabei um benigne Tumoren, die sich als Kondylome, Warzen oder Papillome darstellen, die in der Regel nach Monaten bis Jahren spontan abheilen. Mit Hilfe der Molekularbiologie und Gentechnologie konnte auch der Zusammenhang von verschiedenen Krebserkrankungen mit Papillomvirusinfektionen nachgewiesen werden. Bei diesen Tumoren handelt es sich vor allem um Karzinome der Anogenitalregion, insbesondere um Gebärmutterhalskarzinome, aber auch um Hautkarzinome bei immunsupprimierten Patienten oder Patienten mit der seltenen Erkrankung Epidermodysplasia verruciformis (EV). Die kausale Beteiligung der HPV bei den Krebserkrankungen im Bereich des Anogenitaltraktes gilt mittlerweile als allgemein akzeptiert. Weltweit wird in praktisch allen Gebärmutterhalskarzinomen und Vorläuferläsionen DNA des HPV nachgewiesen.

Mittlerweile wurden mehr als 100 HPV-Genotypen identifiziert, wobei die klinischen Krankheitsbilder, die durch die Infektion verursacht werden können, häufig charakteristisch für einen bestimmten HPV-Typ sind. Vulgäre Warzen der Extremitäten und Plantarwarzen werden vor allem durch die HPV-Typen 1, 2, 4, 10, 27, 28, 57 und 65 verursacht. Die HPV, die im Zusammenhang mit Krebserkrankungen im Anogenitalbereich stehen, werden entsprechend ihres onkogenen Potentials in sogenannte "low risk"-(z.B. 6, 11, 40) und "high risk"-HPV-Typen (z.B. 16, 18, 45 und 56) unterteilt. Low risk-HPV-Typen verursachen hautsächlich gutartige Tumoren des äußeren Genitalbereichs (Kondylome) sowie niedriggradige intraepitheliale Neoplasien und sind nur selten mit Karzinomen assoziiert. Die Anwesenheit von high risk-Viren in Epithelzellen des Anogenitaltraktes stellt hingegen einen hohen Risikofaktor für die Entstehung eines Karzinoms dar. Bei der Entstehung von Hautkarzinomen bei EV-Patienten gilt die Beteiligung der HPV-Typen 5 und 8 als weitgehend gesichert.

Sowohl beim Gebärmutterhalskrebs als auch bei bestimmten Formen des Hautkrebs handelt es sich um vermeidbare Erkrankungen, wenn frühzeitige Erkennung und Behandlung gesichert sind. Eine zuverlässige Methode zum Nachweis einer vorliegenden HPV-Infektion, durch die sämtliche HPV-Typen erfaßt werden und die darüber hinaus eine Typisierung ermöglicht, ist deshalb Voraussetzung für eine zielgerichtete Therapie.

Der Nachweis präkanzeröser Läsionen und frühinvasiver Karzinome erfolgt bereits seit den 50er Jahren mittels histologischer Methoden (Zytologie-Screening). Die Anwendung dieser Methoden hatte zur Folge, dass die Inzidenz von Gebärmutterhalskrebs zurückging. Trotzdem ist die Zytologie methodologisch unzulänglich, was sowohl zu falsch negativen (invasive Karzinome 15 % bis 55 %, frühinvasive Karzinome 20 % bis 70 %) als auch falsch positiven Ergebnissen führt (5 % bis 15 % mit der Folge unnötiger Verängstigung der Patientinnen). Diese Unzulänglichkeiten sind:
- Subjektive Fehler - die Zytologie ist komplex, sie erfordert intensive visuelle Überprüfung von Zellen. Diese Technik ist weder objektiv noch geographisch standardisiert.
- Fehlerhafte oder mangelhafte Probengewinnung kann zu falsch negativen oder unklaren Ergebnissen führen.
- Durch histologische Verfahren wird generell nur der momentane Zustand eines Gewebes erfaßt, so dass der prognostische Wert dieser Methode fehlt.

Durch verbesserte Nachweismethoden ist es inzwischen gelungen, in infizierten Geweben direkt das Vorhandensein des Papillomvirus nachzuweisen:

Bei der sogenannten HCM-Methode (Hybride Capture Microplate), einem von der Firma Digene Corp., Gaithersburg, MD, USA, kommerziell zu beziehendem Test, handelt es sich um ein signalverstärkendes Hybridisierungsverfahren. Damit gelingt der qualitative Nachweis von 18 HPV-Typen in Gewebeproben des Gebärmutterhales. Als Hybridisierungssonden werden HPV-spezifische RNA-Sequenzen verwendet, die das vollständige Virusgenom abdecken.

Nach Inkubation dieser Sonden mit denaturierter HPV-DNA aus dem infizierten Gewebe bilden sich RNA/DNA-Hybride, die mittels eines spezifischen Antikörpers detektiert werden können. Über das Enzym Alkalische Phosphatase, das mit dem Antikörper konjugiert ist, erfolgt nach Zugabe eines entsprechenden Substrates der Nachweis mittels Chemilumineszenz.

Die HCM-Methode ermöglicht die Differenzierung zwischen zwei HPV-DNA-Gruppen: den HPV-Typen 6, 11, 42, 43, 44 mit niedrigem kanzerogenem Potential und den HPV-Typen 16, 18, 31, 33, 35, 39, 45, 51, 52, 56, 58, 59, 68 mit mittlerem bis hohem kanzerogenem Potential.

Eine genaue Typisierung der Papillomviren ist mit dieser Methode nicht möglich. Nachteilig ist bei dieser Testmethode des weiteren, dass es aufgrund der Verwendung eines RNA-Gemisches häufig zu Kreuzreaktionen zwischen beiden HPV-Klassen kommt. Dies kann dann zu falsch negativen oder falsch positiven Ergebnissen führen. Nachteilig ist auch, dass die Papillomviren HPV 5 und 8 mit dieser Methode nicht erfaßt werden.

Surentheran et al., "Detection and typing of Human Papilloma Viruses in mucosal and cutaneous biopsis from immunosuppressed and immunocompetent patients and patients with Epidermodysplasia verruciformis: A unified diagnostic approach", Journal of Clinical Pathology" 1998; 51:606-610, beschreiben ein Polymerase-Kettenreaktion (PCR)-gestütztes Verfahren zum Nachweis von HPV-DNA in Haut- und Schleimhautbiopsien aus immunsupprimierten und immunkompetenten und EV-Patienten. Die Autoren verwenden hierzu verschiedene PCR-Primer-Paare, die alle innerhalb des offenen Leserasters des viralen Gens für das Strukturprotein L1 liegen.

Eine Typisierung der Papillomviren erfolgt durch anschließende Sequenzanalyse des amplifizierten Genfragments. Neuere Untersuchungen haben ergeben, dass das L1-Gen über alle bekannten HPV-Typen weniger konserviert ist als ursprünglich angenommen. Deshalb gelingt mit dieser Methode lediglich der Nachweis einer begrenzten Anzahl von HPV-Typen (HPV 2 - 6, 8, 10, 11, 16, 31, 41, 57). Ob der Nachweis der high risk-HPV-Typen 18, 45 und 56 mit dieser Methode möglich ist, ist nicht beschrieben.

Auch Jacobs et al., "Group-Specific Differentiation between High- and Low-Risk Human Papillomavirus Genotypes by General Primer-Mediated PCR and Two Cocktails of Oligonucleotide Probes", Journal of Clinical Microbiology 1995, 33, 901-905, verwenden ein GP5+/GP6+ bezeichnetes Primer-Paar, das innerhalb des viralen Gens für das Strukturprotein L1 liegt. Der in der PCR mit diesem Primer-Paar amplifizierte Bereich des L1-Gens beträgt 150 Basenpaare (bp). Mittels spezifischer Sonden können die Amplifikate nach gelelektrophoretischer Auftrennung den High Risk-HPV-Typen 16, 18, 31, 33, 35, 39, 45, 51, 52, 54, 56 und 58 sowie den Low Risk-HPV-Typen 6, 11, 34, 40, 42, 43 und 44 zugeordnet werden. Die spezifischen Hybridisierungssonden haben jeweils eine Länge von 30 Basen.

Tieben et al., "Detection of Epidermodysplasia verruciformislike human papillomavirus types in malignant and premalignant skin lesions of renal transplant recipients", British Journal of Dermatology 1994; 131, 226-230, beschreiben eine ebenfalls PCR-gestützte Nachweismethode von HPV-DNA in Hauttumoren von Patienten nach einer Nierentransplantation. Die hier verwendeten, verschiedenen PCR-Primer-Paare liegen innerhalb des konservierten Virus-spezifischen E1-Gens, das für eine ATP-abhängige DNA-Helikase kodiert. Durch die Verwendung von vier verschiedenen PCR-Primer-Paaren gelingt den Autoren ein Erfassen folgender HPV-Typen: HPV 1 - 5, 6b, 7 - 9, 10a, 11, 12, 14a, 15, 16 - 22, 24, 25, 31, 33, 36 - 38, 46, 49, 50, 65. Der Erfinder der vorliegenden Anmeldung hat allerdings herausgefunden, dass das Primer-Paar CP4/CP5 nicht zur Amplifikation geeignet ist.

Eine Typisierung erfolgt bei dieser Veröffentlichung über die Sequenzierung des Amplifikates. Bemerkenswert ist hier, dass lediglich drei der obigen Primer-Paare verwendet werden können, wenn sich solch eine Typisierung an die PCR anschließen soll. Nicht verwendet werden kann das oben erwähnte Primer-Paar CP4/CP5. Mit dieser Methode gelingt Tieben et al. durch Typisierung der spezifische Nachweis der HPV-Typen 1a, 2a, 3 - 5, 6b, 8, 10a, 11 - 13, 14a, 15 - 18, 20 - 22, 24, 25, 31, 33, 36, 39, 41, 42, 46, 47, 49, 50, 57, 58, 63 und 65.

Der Vorteil gegenüber der von Surentheran et al. beschriebenen Methode liegt darin, dass hier mit einem Primer-Paar eine größere Anzahl an verschiedenen HPV-Typen bestimmt werden kann. Trotz dieser verbesserten Spezifität ist die Typisierung vieler HPV, insbesondere der high risk-Papillomviren HPV 45 und 56 sowie der HPV-Typen mit mittlerem onkogenem Potential 35, 51 und 52 mit dieser Methode nicht möglich.

Nach Kenntnis des Erfinders der vorliegenden Anmeldung wird das Primer-Paar CP4/CP5 jedoch im Klinikalltag nicht eingesetzt, hier finden z. Zt. vielmehr nur Primer-Paare Anwendung, die innerhalb des L1-Gens liegen, wie z.B. das von Jacobs et al., a.a.O. beschriebene Primer-Paar GP5+/GP6+.

Vor diesem Hintergrund ist es Aufgabe der vorliegenden Erfindung, ein Verfahren der eingangs genannten Art zu schaffen, das einen zuverlässigen Nachweis von allen derzeit im Zusammenhang mit der Tumorinduktion in Frage kommenden HPV-Viren ermöglicht, sowie Hybridisierungs-Sequenzen für selbiges Verfahren bereitzustellen.

Bei dem eingangs genannten Verfahren wird diese Aufgabe erfindungsgemäß dadurch gelöst, dass als Primer-Paar für die PCR Oligonukleotide verwendet werden, welche mit Oligonukleotiden hybridisieren, mit welchen eines der Oligonukleotide mit der Sequenz SEQ ID Nr. 1, SEQ ID Nr. 2 oder SEQ ID Nr. 3 hybridisiert, und welche Oligonukleotide die gleiche Funktion aufweisen wie die Oligonukleotide mit der SEQ ID Nr. 1, SEQ ID Nr. 2 und SEQ ID Nr. 3 aus dem beiliegenden Sequenzprotokoll.

Die der Erfindung zugrunde liegende Aufgabe wird auf diese Weise vollkommen gelöst. Der Erfinder der vorliegenden Anmeldung hat nämlich erkannt, dass durch den Einsatz der hier erstmals beschriebenen Nukleotidsequenzen SEQ ID Nr. 1, 2 und 3 aufgrund ihrer sehr hohen Spezifität und Sensitivität der Nachweis aller bisher bekannten HPV-Typen gelingt. Alle drei Sequenzen stammen aus dem hochkonservierten viralen E1-Gen. Der Einsatz der Sequenzen SEQ ID Nr. 1, 2 und 3, z.B. als Hybridisierungssonden (DNA-Marker) oder im Rahmen einer DNA-Amplifizierungsmethode eignet sich deshalb besonders für den Nachweis einer allgemeinen HPV-Infektion.

Hierzu werden z.B. die Oligonukleotide, die mit Oligonukleotiden hybridisieren, mit welchen eines der Oligonukleotide mit der Sequenz SEQ ID Nr. 1, SEQ ID Nr. 2 oder SEQ ID Nr. 3 hybridisiert, und welche Oligonukleotide die gleiche Funktion aufweisen wie die Oligonukleotide mit der SEQ ID Nr. 1, SEQ ID Nr. 2 und SEQ ID Nr. 3 aus dem beigefügten Sequenzprotokoll. Dabei entsteht ein Amplifikationsprodukt von ca. 220 bp, das mittels einfacher Standardmethoden, z.B Ethidiumbromidanfärbung nach gelelektrophoretischer Auftrennung, nachgewiesen werden kann.

Der Einsatz der Sequenz SEQ ID Nr. 1 zusammen mit der Sequenz SEQ ID Nr. 3 als PCR-Primer-Paar liefert ein Amplifikationsprodukt des viralen E1-Gens von ca. 280 bp Länge. Dies ermöglicht aufgrund der Länge des Amplifikats (im Vergleich zum 220 bp-Amplifikat bei Verwendung von SEQ ID Nr. 1 und SEQ ID Nr. 2 als PCR-Primer-Paar) eine noch sicherere Typisierung, z.B. durch anschließende Sequenzierung oder Temperaturgradientengelelektrophorese, als bei Verwendung des Primer-Paares SEQ ID Nr. 1 und 2.

Ein weiterer Vorteil ist darin zu sehen, daß durch die Beschaffenheit der Nukleotidsequenz SEQ ID Nr. 1, die in keiner Position degeneriert ist, eine eventuelle Sequenzierung des Amplifikationsproduktes erleichtert wird. Die von Tieben et al. verwendeten Primer sind dagegen in zwei bzw. drei Positionen degeneriert. Das hat zur Folge, daß in der PCR sehr hohe Primermengen eingesetzt werden müssen und bei eventuell anschließendem Sequenzieren erhöht Probleme auftreten.

Es ist auch möglich, die beschriebenen Primer-Paare in einem Verfahren für quantitative PCR einzusetzen, das auf dem sogenannten LightCycler^{TM} von Roche Molecular Biochemicals durchgeführt werden kann. Der Nachweis der amplifizierten DNA kann dabei sowohl über den für den Nachweis von doppelsträngiger DNA verwendeten Farbstoff CYBER™Green I als auch über sequenzspezifische DNA-Sonden erfolgen, die mit verschiedenen Farbstoffen markiert sind.

Ein bevorzugtes Ausführungsbeispiel der vorliegenden Erfindung besteht darin, dass die Amplifikation der spezifischen HPV-DNA-Abschnitte mittels Polymerase-Kettenreaktion (PCR) erfolgt, die durch folgendes Temperaturprofil charakterisiert ist:

| | |
|---|---|
| Aufheizen: | 5 - 15 min., 90 - 100 °C; |
| Hitzedenaturierung: | 30 - 90 sec., 90 - 100 °C; |
| Hybridisierung: | 30 - 90 sec., 50 - 60 °C; |
| Polymerisierung: | 30 - 180 sec., 67 - 77 °C; |
| Abkühlen: | 2 - 10 min., 67 - 77 °C. |

Der Erfinder der vorliegenden Anmeldung hat erkannt, dass dieses Temperaturprofil, das von anderen publizierten Profilen abweicht, für eine effiziente Amplifizierungsreaktion und damit zuverlässige Typisierung wesentlich ist. Eine besonders effiziente PCR-Reaktion wird nach Erkenntnissen des Erfinders bei folgenden Bedingungen erreicht:

| | |
|---|---|
| Aufheizen: | 10 min., 95 °C; |
| Hitzedenaturierung: | 30 sec., 95 °C; |
| Hybridisierung: | 30 sec., 55 °C; |
| Polymerisierung: | 1 min., 72 °C; |
| Abkühlen: | 5 min., 72 °C; |
| Aufheizrate: | 1 °C/sec; |
| Zyklen: | 40. |

Eine besonders zuverlässige Typisierung der HPV wird durch Sequenzierung der amplifizierten HPV-DNA-Abschnitte erzielt. Das Amplifikationsprodukt ist nämlich durch geringe Sequenzvariationen zwischen den verschiedenen HPV-Typen charakterisiert, was die Zuordnung des Typs ermöglicht.

Das beschriebene Verfahren wird bevorzugt als Teil eines Verfahrens zur Diagnose und/oder Früherkennung eingesetzt, insbesondere im Zusammenhang mit Krebserkrankungen, beispielsweise dem Gebärmutterhalskrebs.

Der Erfinder hat hier herausgefunden, dass diese Methode, deren Bestandteile in anderen Zusammenhängen bisher überwiegend im Forschungsbereich eingesetzt wurden, ein zuverlässiges Diagnoseverfahren darstellt.

Das neue Verfahren ist ebenfalls dazu geeignet, im Rahmen einer Früherkennungsuntersuchung von Krebserkrankungen eingesetzt zu werden. Nur ein gesicherter positiver Befund hinsichtlich einer HPV-Infektion kann nämlich Ausgangspunkt für eine zielgerichtete Therapie sein.

Gegenstand der Erfindung sind auch die in dem Verfahren verwendeten Nukleotidsequenzen SEQ ID Nr. 1, 2 und 3 gemäß beigefügtem Sequenzprotokoll, sowie Nukleotidsequenzen, die an Sequenzen hybridisieren, an die die Sequenzen SEQ ID Nr. 1, SEQ ID Nr. 2 oder SEQ ID Nr. 3 binden, und die die gleiche Funktion haben wie die Oligonukleotidsequenzen mit der SEQ ID Nr. 1, SEQ ID Nr. 2 oder SEQ ID Nr. 3.

Durch Verkürzung, Verlängerung, Substitution, Insertion und/oder Deletion können die Oligonukleotidsequenzen mit der SEQ ID Nr. 1, SEQ ID Nr. 2 oder SEQ ID Nr. 3 verändert werden zu Oligonukleotiden, jedoch immer noch funktionell vergleichbar sind. Vor diesem Hintergrund betrifft die Erfindung auch derart veränderte Nukleotidsequenzen.

Selbiges gilt für Nukleotidsequenzen, die mindestens zu einer der vorstehenden Sequenzen komplementär sind.

Der Erfinder hat auch erkannt, dass die Verwendung komplementärer Nukleotidsequenzen, die entsprechend am Gegenstrang der HPV-DNA hybridisieren, in dem oben beschriebenen Verfahren zu gleichen Resultaten führen kann. Deshalb sind solche Sequenzen ebenfalls Bestandteil der vorliegenden Erfindung.

Eine besondere Leistung des Erfinders liegt in der Erkenntnis, dass eine Nukleotidsequenz aus dem offenen Leseraster E1 des HPV zum Nachweis und sogar zur Typisierung von HPV im Anogenitalbereich, insbesondere im Zusammenhang mit einer Gebärmutterhalserkrankung, verwendet werden kann, weil die E1-Region im Vergleich zur L1-Region über sämtliche bekannten HPV-Typen eine höhere Konservierung zeigt.

Die hohe Konservierung des viralen E1-Gens gegenüber dem viralen L1-Gens sichert die Erfassung möglichst vieler HPV-Typen. Trotz allem vorhandene, geringfügige Sequenzvariationen zwischen den verschiedenen HPV-Typen gewährleisten wider Erwarten auch eine zuverlässige Typisierung.

Es versteht sich, dass die vorstehend erwähnten Merkmale nicht nur in der angegebenen Kombination, sondern auch einzeln oder in anderer Kombination verwendbar sind, ohne den Rahmen der vorliegenden Erfindung zu verlassen.

Die Erfindung wird nachstehend anhand von Beispielen erläutert, aus denen sich weitere Merkmale und Vorteile ergeben.

### Beispiel 1: DNA-Extraktion

Für den Nachweis von HPV ist es zunächst erforderlich, Biopsie-oder Abstrichmaterial zu entnehmen. Dieses wird in 500 µl Melting-Puffer (0,1 M EDTA; 0,05 M Tris pH 8,0; 0,5 % SDS) aufgenommen. Dazu werden 2,5 µl Proteinase K (20 mg/ml) gegeben und die Lösung für 24 bis 48 Stunden bei 55° C inkubiert.

Daran schließt sich eine zweimalige Phenol-Extraktion (Phenol:CHC1₃:Isoamylalkohol = 25:24:1) an. Der Ansatz wird gemischt, nicht gevortext, für 2 - 5 min. inkubiert (über Kopf rotiert), danach bei 14000 rpm für 5 min. abzentrifugiert. Es folgt eine Extraktion mit CHCl₃/Isoamylalkohol (24:1), Inkubation für 2 - 5 min., danach 5 min. bei 14000 rpm zentrifugieren.

Es folgt die Zugabe von 1/10 des Ansatzvolumens an 7,5 M Ammoniumacetat, der Ansatz wird gemischt und 2 bis 4 Std. bei 55 °C inkubiert.

Der Ansatz wird dann über Nacht bei -20 °C ethanolgefällt.

Die so extrahierte DNA wird anschließend in eine PCR-Reaktion eingesetzt.

### Beispiel 2: PCR-Reaktion

Die PCR dient der gezielten Amplifikation viraler DNA-Abschnitte.

Hierzu werden 0,25 µl, 1 µl, 2,5 µl und 5 µl der extrahierten DNA-Menge eingesetzt (ohne Konzentrationsbestimmung); Zusatz Primer 1: 100 ng, Primer 2: 100 bzw. 200 ng, dNTPs: je 10 mM; 5 µl Taq-Polymerase-Puffer (10-fach, ohne MgC1₂); 7 µl MgC1₂ (25 mM Stammlösung, entspricht 3,6 mM); 0,4 µl Taq-Gold-Polymerase (entspricht 2 units). Der Ansatz wird auf ein Gesamtvolumen von 50 µl aufgefüllt. Die Durchführung der PCR erfolgt in einem MJ-Research Thermocycler (PTC 200); eingestellte Aufheizrate: 1 °C/sec. Die PCR-Amplifikation läuft über 40 Zyklen [10 min. 95 °C (Aufheizen, nur im ersten Zyklus); 30 sec. 95 °C; 30 sec. 55 °C; 1 min. 72 °C; 5 min. 72 °C (Abkühlen, nur im letzten Zyklus)].

Zum unspezifischen Nachweis einer HPV-Infektion (Screening) werden bevorzugt die Sequenzen SEQ ID Nr. 1 (Vorwärts) und SEQ ID Nr. 2 (Rückwärts) als Primer-Paar (je 100 ng) verwendet. Die Nukleotidsequenzen dieser Primer und die Lage im HPV-Genom (HPV16 als Beispiel) sind:

SEQ ID Nr. 1 5'-(nt2082)-AAC-AAT-GTG-TAG-ACA-TTA-TAA-
ACG-AGC-(nt2108)-3'

SEQ ID Nr. 2 5'-(nt2336)-ATT-AAA-CTC-ATT-CCA-AAA-TAT-
GA-(nt2314)-3'

Durch dieses Screening-Verfahren werden derzeit alle bekannten HPV-Typen erfaßt.

Soll sich eine Typisierung, z.B. mittels DNA-Sequenzierung, anschließen, verwendet man bevorzugt die Sequenzen SEQ ID Nr. 1 (Vorwärts) und SEQ ID Nr. 3 (Rückwärts; 200 ng) als Primer-Paar: 
SEQ ID Nr. 3:          5'-(nt2401)-GAG-GYT-GCA-ACC-AAA-AMT-GRC-
                       T-(nt2380)-3',
wobei Y für ein Pyrimidin steht (T oder C), M Amino bedeutet (A oder C) und R für ein Purin steht (G oder A).

Bei den Rückwärts-Primern SEQ ID Nr. 2 und 3 sind die Positionen des antikodierenden DNA-Stranges angegeben, zu dem diese Primer reverse und komplementär sind.

### Beispiel 3: DNA-Sequenzierung (Typisierung)

Die Sequenzierung des Amplifikats aus Beispiel 2 zur Bestimmung des HPV-Typs wird mit dem Big Dye Terminator Cycle Sequencing Ready Reaction Kit mit Ampli-Taq von der Firma ABI unter Verwendung von 200 ng Primer SEQ ID Nr. 1 oder 3 und des PCR-Amplifikationsansatzes durchgeführt. Dieser wird zuvor über Microcon 100 Säulen (Millipore) eingeengt und aufgereinigt. Von dem aufgereinigten Ansatz werden ca. 50 % für die Sequenzierung eingesetzt.

Die Sequenzier-PCR-Reaktion wird nach den folgenden Bedingungen durchgeführt: 10 sec. 96 °C, 5 sec. 55 °C, 4 min. 60 °C, 30 Zyklen, mit einer Aufheizrate von 1 °C/ sec.

Gefällt wird anschließend mit 0,3 M Natriumacetat und 2,5-fachem Volumen Ethanol.

Das Pellet wird in 25 µl TSR (Template Suppression Reagent von ABI) aufgenommen, für 2 min. bei 90 °C inkubiert, danach für 2 min. auf 4 °C gebracht, und anschließend auf Raumtemperatur gehalten.

Die Kapillarelektrophorese mit Hilfe des ABI-Prism 310 Sequenzierautomaten wird mit einer 47 cm Kapillare (Durchmesser 50 µm) durchgeführt, und mit dem 310 Genetic Analyser-Programm von ABI ausgewertet. Als Polymer wird das Polymer POP 6 (Performance Optimize Polymer 6) von ABI verwendet.

Durch diese Typisierung können bei Verwendung der Primer SEQ ID Nr. 1 und SEQ ID Nr. 3 folgende HPV-Typen identifiziert werden:

| | |
|---|---|
| HPV1 bis HPV8 | HPV51 bis HPV54 |
| HPV10 bis HPV19 | HPV56 bis HPV60 |
| HPV21 bis HPV28 | HPV65 bis HPV67 |
| HPV30 bis HPV38 | HPV70 |
| HPV40 | HPV72 bis HPV73 |
| HPV42 | HPV82 bis HPV83 |
| HPV44 bis HPV47 | |

Bei Verwendung der PCR-Primer-Paare SEQ ID Nr. 1 und SEQ ID Nr. 2 werden analoge Ergebnisse erzielt. Aufgrund des kleineren Amplifikationsproduktes sind folgende HPV-Typen nicht zu differenzieren:
HPV51/HPV31
HPV31/HPV70
HPV18/HPV6
HPV18/HPV2a
HPV33/HPV1
HPV31/HPV83
HPV31/HPV57

### Beispiel 4: Verwendung einer Nukleotidsequenz aus dem offenen Leseraster E1 des HPV

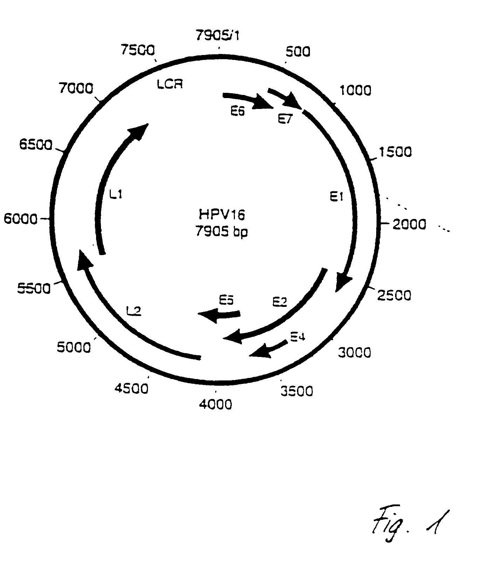

Das Genom des HPV ist in der Fig. 1 beispielhaft anhand des 7.905 bp langen HPV16-Genoms dargestellt. Das Genom gliedert sich in drei Abschnitte: eine nicht kodierende Region (LCR, Long Control Region), eine E-(early-)Region, die für die "frühen Gene" kodiert und eine L-(late-)Region, die für die "späten Gene" kodiert. Die Lage der offenen Leseraster ist anhand der Positionsangaben ersichtlich.

Aus dem offenen Leseraster E1, das für eine ATP-abhängige DNA-Helikase kodiert, werden erfindungsgemäß eine oder mehrere Nukleotidsequenzen zum Nachweis von HPV in der Art verwendet, dass hiermit sämtliche HPV-Typen erfaßt werden. Der Einsatz solcher Sequenzen ist in Form von Hybridisierungssonden oder auch von PCR-Primern etc. möglich. Hier kommt besonders eine HPV-Infektion im Anogenitalbereich, insbesondere im Zusammenhang mit einer Gebärmutterhalserkrankung, in Betracht.

Die E1-Region ist durch eine hohe Konservierung innerhalb der bekannten HPV-Typen gekennzeichnet. Deshalb ist die E1-Region besonders geeignet, eine allgemeine HPV-Infektion nachzuweisen. Es hat sich herausgestellt, dass es bei einer Verwendung des offenen Leserasters L1 zum Nachweis von HPV aufgrund von größeren Sequenzvariationen zwischen den verschiedenen HPV-Typen zu falsch negativen Ergebnissen kommen kann. Dieses Risiko des fehlerhaften Nachweises wird durch die Verwendung der E1-Region minimiert.

Dennoch kann die E1-Region auch zur Typisierung des HPV eingesetzt werden, die geringen Sequenzunterschiede reichen dann nach Erkenntnis des Erfinders überraschenderweise aus.

### Beispiel 5: Quantitative PCR mit dem Primer-Paar SEQ ID Nr. 1 und SEQ ID Nr. 3

Mit Hilfe des LightCyclers™ wird das Genom von HPV33 unter Einsatz des Primer-Paares SEQ ID Nr. 1 und SEQ ID Nr. 3 nachgewiesen. Hierzu wird nach Angaben des Herstellers der Zehnfach LC-DNA Master Hybridization Fast-Start Probe-Mix (ohne MgCl₂) eingesetzt und die MgCl₂-Entkonzentration auf 4 mM eingestellt.

Die eingesetzte DNA-Menge von HPV33 betrug 30 ng, die beiden Primer konnten jeweils im Bereich von 0,45 bis 0,9 µM verwendet werden.

Die PCR-Amplifikation erfolgte nach folgendem Protokoll:
- Initiale Denaturierung bei 95°C, 10 Minuten
- Amplifikation (50 Zyklen) mit jeweils 95°C, 10 Sekunden, 55°C, 15 Sekunden und 72°C, 15 Sekunden. Die Abkühlung erfolgte bei 40°C, 1 Minute.

Die Aufheiz-Raten betrugen in allen bis auf einen Schritt jeweils 20°C/Sekunde, in dem 72°C-Schritt aus der Amplifikation jeweils mit 5°C/Sekunde.

Am Ende des Annealings erfolgte die Fluoreszenzmessung.

Mit Hilfe des an doppelsträngige DNA bindenden Farbstoffes CYBER™GREEN I konnte im Vergleich zu einem HPV-Plasmidstandard eine Empfindlichkeit dieses Verfahrens nachgewiesen, die noch einen Nachweis von lediglich zehn Kopien an HPV-Genom pro Probe ermöglicht.

In einem weiteren Test wurden zwei sequenzspezifische DNA-Sonden verwendet, die mit verschiedenen Farbstoffen markiert wurden. Die Sequenzen der beiden Sonden sind so ausgewählt, dass sie so an die Zielsequenz des verstärkten DNA-Segmentes hybridisiert sind, dass das 3'-Ende der einen Sonde dicht bei dem 5'-Ende der anderen Sonde liegt, wobei die beiden Farbstoffe nahezu einander gebracht werden. Der eine Farbstoff ist ein Donor-Farbstoff und wird durch eine kurzwellige Lichtquelle angeregt, woraufhin er Fluoreszenzlicht bei einer etwas längeren Wellenlänge emittiert. Wenn sich die beiden Farbstoffe dicht nebeneinander befinden, regt die emittierte Energie des Donor-Farbstoffes den Akzeptor-Farbstoff an, der an der zweiten Hybridisierungssonde sitzt und ein Fluoreszenzlicht bei einer anderen Wellenlänge emittiert.

Für den Nachweis von HPV33 wurden die beiden folgenden Sonden eingesetzt:

3'-Sonde:     CGTAAAATGT CAATAGGACA ATGGATACAA

5'-Sonde:     AGATGTGAAA AAACAAATGA TGGAGGAAA

Die 3'-Sonde wurde mit dem Farbstoff LC-Red640 und die 5'Sonde mit dem Farbstoff FITC markiert. Beide Sonden wurden im Bereich von 0,15 bis 0,25 µM eingesetzt.

Die PCR-Amplifikation erfolgte nach dem Protokoll des Herstellers, auch hier konnte eine erreichbare Sensitivität von 10 Genom-Kopien pro eingesetzter Probe gezeigt werden.

### Beispiel 6: Vergleich zwischen den Primer-Paaren GP5+/GP6+ und SEQ ID Nr. 1/SEQ ID Nr. 3 sowie dem Hybrid Capture Verfahren

Mit dem Hybrid Capture II HPV DNA Test der Firma Digene Corporation, Gaithersburg, MD, USA, wurden verschiedene Zervikalabstriche auf HPV untersucht, es ergaben sich 15 High Risk HPVpositive und 24 High Risk HPV-negative Proben.

Diese Proben wurden unter Verwendung des Primer-Paares SEQ ID Nr. 1 und SEQ ID Nr. 3 und mit der in Beispiel 2 gezeigten PCR-Reaktion ebenfalls auf HPV überprüft. Parallel wurden diese Proben auch mit dem GP5+/GP6+ Primer-Paar und den in der Veröffentlichung von Jacobs et al. a.a.O. angegebenen Reaktionsbedingungen überprüft.

Von den 15 HCII High Risk HPV-positiven Proben waren mit GP5+/GP6+ elf Proben positiv, mit SEQ ID Nr. 1/SEQ ID Nr. 3 jedoch alle 15 Proben positiv.

Von den 24 HCII High Risk HPV-negativen Proben waren mit GP5+/GP6+ noch zwei Proben positiv, mit SEQ ID Nr. 1/SEQ ID Nr. 3 sogar sieben Proben positiv.

In allen Fällen mit positivem Befund für SEQ ID Nr. 1/SEQ ID Nr. 3 konnte bis auf einen der vorliegende HPV-Typ durch direkte Sequenzierung der Amplifikate bestimmt werden. Bei den insgesamt 13 positiven GP5+/GP6+ Proben gelang dies nur in sieben Fällen, da sich bei Patientenproben im Gel Mehrfachbanden zeigten, was auf unspezifische Amplifikate zellulärer Gene hinzuweisen scheint. Von diesen sieben Fällen wich das Ergebnis in zwei Fällen von dem Ergebnis mit SEQ ID Nr. 1/SEQ ID Nr. 3 ab.

Die hier mittels SEQ ID Nr. 1/SEQ ID Nr. 3 gefundenen HPV-Typen waren: 2a, 16, 18, 31, 32, 33, 35, 42, 45, 51, 53, 72.

Diese Vergleichsversuche zeigen, dass das Primer-Paar SEQ ID Nr. 1/SEQ ID Nr. 3 deutlich sensitiver ist als das Hybrid Capture Verfahren oder ein Verfahren, bei dem die Sonden GP5+/GP6+ eingesetzt werden. GP5+/GP6+ zeigte im Vergleich zu SEQ ID Nr. 1/SEQ ID Nr. 3 neun falsch-negative Proben, während das Hybrid Capture Verfahren verglichen mit SEQ ID Nr. 1/SEQ ID Nr. 3 sieben falsch-negative Proben zeigte.

## Patentansprüche

1. Oligonukleotid, das mit Oligonukleotiden hybridisiert, mit welchen eines der Oligonukleotide mit der Sequenz SEQ ID Nr. 1, SEQ ID Nr. 2 oder SEQ ID Nr. 3 hybridisiert, und welches die gleiche Funktion aufweist wie die Oligonukleotide mit der SEQ ID Nr. 1, SEQ ID Nr. 2 und SEQ ID Nr. 3.

2. Verfahren zum Nachweis von Humanen Papillomviren (HPV) in biologischem Material, mit den Schritten:
a) Extraktion/Isolation von Nukleinsäuren aus dem biologischen Material, und
b) Nachweis HPV-spezifischer DNA aus den isolierten Nukleinsäuren über eine Amplifikation spezifischer HPV-DANN-Abschnitte mittels Polymerase-Kettenreaktion (PCR),
**dadurch gekennzeichnet, dass** als Primer-Paar für die PCR Oligonukleotide gemäß Anspruch 1 verwendet werden.

3. Verfahren nach Anspruch 2, **dadurch gekennzeichnet, dass** die PCR durch folgendes Temperaturprofil charakterisiert ist:
| | |
|---|---|
| Aufheizen: | 5 - 15 min., 90 - 100 °C; |
| Hitzedenaturierung: | 30 - 90 sec., 90 - 100 °C; |
| Hybridisierung: | 30 - 90 sec., 50 - 60 °C; |
| Polymerisierung: | 30 - 180 sec., 67 - 77 °C; |
| Abkühlen: | 2 - 10 min., 67 - 77 °C. |

4. Verfahren nach Anspruch 2 oder 3, **dadurch gekennzeichnet, dass** eine Typisierung der HPV durch Sequenzierung der amplifizierten HPV-DNA-Abschnitte erfolgt.

5. Verfahren nach einem der Ansprüche 2 bis 4, **dadurch gekennzeichnet, dass** dieses Teil eines diagnostischen Verfahrens und/oder Früherkennungsverfahrens ist.

6. Verfahren nach Anspruch 5, **dadurch gekennzeichnet, dass** dieses Teil eines Verfahrens zur Diagnose und/oder Früherkennung von Krebserkrankungen, insbesondere von Gebärmutterhalskrebs, ist.
